# FASCICULE DE BREVET EUROPEEN

(11) **EP 2 739 321 B1**
(45) Date de publication et mention de la délivrance du brevet: **29.04.2015**
(21) Numéro de dépôt: 12750449.6
(22) Date de dépôt: 26.07.2012
(51) Int. Cl.: A61L 9/04, A61L 9/12

(54) **CAPSULE DE PARFUM ET DISPOSITIF DE DIFFUSION DE PARFUM ASSOCIE**
DUFTSTOFFKAPSEL UND ZUGEHÖRIGE DUFTSTOFFVERBREITENDE VORRICHTUNG
FRAGRANCE CAPSULE AND ASSOCIATED FRAGRANCE-DIFFUSING DEVICE

(30) Priorité: 05.08.2011 FR 1157183
(43) Date de publication de la demande: 11.06.2014
(73) Titulaire: Presensia, 75002 Paris (FR)
(72) Inventeur: SUISSA, David, F-94300 Vincennes (FR)
(74) Mandataire: Fidal Innovation
(86) Numéro de dépôt international: PCT/FR2012/051781
(87) Numéro de publication internationale: WO 2013/021114

(56) Documents cités:
- WO-A1-2006/029690
- WO-A2-03/105652
- WO-A2-2004/096588
- WO-A2-2008/044201
- JP-A- 2002 291 392
- US-A- 4 968 456
- US-A1- 2002 197 186

## Description

L'invention concerne de manière générale le domaine de la diffusion de parfum et des supports de substances odorantes.

Les supports de substances odorantes peuvent être classés comme suit :
- les réservoirs de substances odorantes à l'état liquide destinées à être vaporisées soit par micronisation exploitant l'effet venturi couplé ou non à des micro vibrations, soit au moyen d'une mèche exploitant l'effet de capillarité soumise à un flux d'air ;
- les matériaux qui passent de l'état solide à l'état liquide puis à l'état de vapeur sous l'effet de la chaleur, le cas le plus classique étant la bougie odorante ;
- les supports humides imbibés de substances, comme les lingettes odorantes ; et
- les supports solides dans lesquels sont adsorbées des molécules odorantes que l'on libère dans l'atmosphère par diffusion spontanée ou à l'aide d'un flux d'air, par exemple certains filtres odorants utilisés pour l'habitacle d'automobile ou des capsules sèches de polymères utilisées dans certains dispositifs de diffusion d'odeurs d'ambiance.

L'invention concerne cette dernière classe de supports et plus particulièrement une capsule de parfum comprenant des éléments de substrat dans lesquels est adsorbé un parfum. L'invention concerne également un dispositif de diffusion de parfum associé.

Concernant l'état de l'art, des capsules sèches de billes en matériau polymère sont décrites par exemple dans le document EP1054697. Selon ce document, une capsule est placée dans un conduit cylindrique pratiqué dans un boisseau de forme globalement sphérique afin de permettre l'isolation de la capsule par rapport à l'environnement par une rotation du boisseau lorsque l'on ne souhaite pas diffuser d'odeur. Aucune précision n'est toutefois apportée dans ce document concernant le positionnement des billes à l'intérieur de la capsule.

Par ailleurs, le document EP0104758 décrit une capsule dotée d'un organe de protection pour l'isoler hermétiquement de l'air ambiant lorsqu'elle n'est pas utilisée. Aucune précision n'est apportée dans ce document concernant le positionnement des billes dans la capsule.

Il en est de même dans les documents FR1500142, WO03105652, WO2009003704 et WO2006046940 qui ne donnent pas d'indication concernant un positionnement particulier des éléments odorants.

Un dispositif d'émission de parfum, comprenant une pluralité de billes parfumées contenues à l'intérieur d'une cage est connu par le document WO-A-2006/029690

Dans ce contexte, la présente invention propose une capsule de parfum qui présente au moins un avantage technique sur les supports précédemment évoqués.

A cette fin, la capsule de parfum comprend :
- des éléments de substrat dans lesquels est adsorbé un parfum,
- un cadre,
- une première grille et une seconde grille fixées dans le cadre en vis-à-vis l'une de l'autre, chacune des grilles comprenant une pluralité de barreaux,
les barreaux d'une même grille étant espacés entre eux et les grilles étant espacées entre elles, de sorte que la capsule permette le maintien dans le cadre et entre les grilles d'une seule couche d'éléments de substrat, et une circulation d'air dans le cadre autour des éléments de substrat et des barreaux.

Que les éléments de substrat soient disposés dans la capsule sur une seule couche présente plusieurs avantages. Premièrement, la surface totale des éléments de substrat au contact de l'air circulant dans le cadre est optimisée, voire maximisée, pour un nombre d'éléments de substrat donné. Deuxièmement, la vitesse à laquelle le parfum est désorbé dans l'atmosphère est sensiblement la même pour chaque élément de substrat. Troisièmement, l'épaisseur de la capsule, ou équivalemment du cadre, est relativement faible en comparaison de ses autres dimensions ce qui rend la capsule compatible avec des dispositifs de diffusion de parfum compacts. Quatrièmement, un flux d'air traversant la capsule est équiréparti sur les éléments de substrat et ainsi une qualité de parfum parfaite est obtenue.

En outre, la capsule étant destinée à être insérée dans un dispositif de diffusion de parfum compact, comprenant au moins un générateur de flux d'air propre à faire circuler de l'air d'amont en aval de la capsule, les avantages suivants sont également atteints. Premièrement, une diffusion satisfaisante par le dispositif des molécules de parfum dans l'atmosphère nécessite une pression moindre que celle qui serait requise si des éléments de substrat étaient disposés sur au moins deux couches, soulageant d'autant les contraintes auxquelles doit satisfaire le générateur de flux d'air. Deuxièmement, le débit en molécules de parfum diffusées est sensiblement invariant, et ainsi, le dosage de la quantité de molécules de parfum diffusées, dans un laps de temps donné et en fonction du volume de la pièce à parfumer, est aisé et précis.

Selon une particularité, chaque élément de substrat présente des dimensions qui varient dans le temps depuis des dimensions initiales jusqu'à des dimensions finales plus petites atteintes lorsque le parfum est entièrement désorbé.

Selon une autre particularité, les première et seconde grilles sont planes et parallèles entre elles, et les barreaux de chaque grille sont rectilignes et parallèles entre eux, de sorte que chaque barreau de la première grille se trouve en vis-à-vis d'un barreau de la seconde grille.

La capsule est ainsi avantageusement de fabrication industrielle aisée. En outre, le flux d'air la traversant est avantageusement proche d'un flux d'air laminaire en aval de la capsule.

Selon une autre particularité, pour chaque grille, la distance entre deux barreaux premiers voisins et l'épaisseur de chaque barreau sont strictement inférieures à la plus petite dimension des éléments de substrat, et l'espacement entre les première et seconde grilles est supérieur à la plus petite dimension initiale des éléments de substrat et inférieur à la plus grande dimension initiale des éléments de substrat.

Avantageusement, les éléments de substrat sont ainsi au moins initialement maintenus enserrés, ou du moins coincés, entre les barreaux des grilles. En outre, à mesure que leurs dimensions rapetissent, les éléments de substrat deviennent au moins partiellement libres de se mouvoir entre les grilles, sans pour autant pouvoir passer entre les barreaux.

Selon une autre particularité, au moins deux barreaux seconds voisins de la première grille et/ou de la seconde grille s'étendent dans l'épaisseur du cadre, de sorte qu'une seule rangée d'éléments de substrat de la couche d'éléments de substrat soit maintenue entre les deux barreaux seconds voisins s'étendant dans l'épaisseur du cadre.

Avantageusement, la couche d'éléments de substrat comprend ainsi au moins une rangée d'éléments de substrat présentant un nombre d'éléments de substrat relativement aisé à maîtriser lors du chargement industriel des éléments de substrat dans la capsule.

Selon une autre particularité, les barreaux seconds voisins s'étendant dans l'épaisseur du cadre sont disposés verticalement de sorte que, les éléments de substrat de la rangée se tassant dans le temps au moins sous l'action de leur poids, la hauteur de la rangée constitue un indicateur visuel d'usure de la capsule.

Ainsi, l'état d'usure de la capsule est aisément et simplement observable.

Selon une première variante, un poids est disposé au dessus de la rangée de billes. L'on s'assure ainsi du tassement de la rangée dans le temps.

Selon une deuxième variante, la capsule 0 comprend un ressort fixé par une première extrémité dans le cadre 2 au niveau de la rangée et une butée fixée à l'autre extrémité du ressort, de sorte que le ressort soit maintenu, quel que soit l'état d'usure des éléments de substrat 1, dans un état de compression entre le cadre 2 et la butée. L'on s'assure ainsi du tassement de la rangée dans le temps que celle-ci soit disposée verticalement ou horizontalement.

Selon une autre particularité, le cadre comprend au moins un élément mâle de fixation sur une face et au moins autant d'éléments femelles de fixation sur l'autre face, chaque élément mâle étant propre à mettre en prise l'un quelconque des éléments femelles, de sorte que plusieurs capsules puissent être jointes ensemble, leurs cadres se juxtaposant dans la direction de leur épaisseur.

Ainsi, la quantité de molécules de parfum diffusées est modulable, notamment en fonction du volume de la pièce à parfumer.

Selon une autre particularité, un joint d'étanchéité à l'air est disposé au niveau de la jonction entre deux cadres juxtaposés.

Le flux d'air traversant les capsules juxtaposées par leurs cadres est ainsi contraint de traverser chaque capsule de la juxtaposition sans perte de charge du flux d'air qui serait due à des fuites d'air entre les cadres des capsules juxtaposées.

L'invention concerne également un dispositif de diffusion de parfum comprenant :
- une capsule telle que particularisée ci-dessus,
- un couloir de ventilation,
- un générateur de flux d'air agencé pour propulser de l'air dans le couloir de ventilation, et
- un support de fixation adapté pour fixer la capsule de parfum dans le couloir de ventilation,
de sorte que l'air propulsé diffuse le parfum.

D'autres caractéristiques et avantages de l'invention ressortiront clairement de la description qui en est faite ci-après, à titre indicatif et nullement limitatif, en référence aux dessins annexés, dans lesquels :
- la figure 1 représente une vue de face de la capsule selon un mode de réalisation ;
- la figure 2 représente une vue en perspective de la capsule représentée sur la figure 1 ; et
- la figure 3 représente une vue en perspective de la capsule ouverte, i.e. sans la seconde grille,
- la figure 4 illustre la façon dont les capsules peuvent être juxtaposées, et
- la figure 5 représente schématiquement un dispositif de diffusion de parfum selon l'invention.

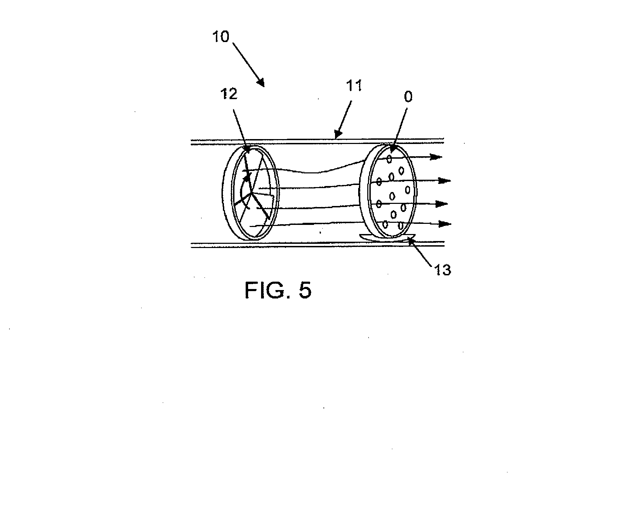

Comme illustré sur la figure 5, la capsule 0 selon l'invention est destinée à être fixée de manière amovible dans un dispositif 10 de diffusion de parfum comprenant :
- un couloir de ventilation 11,
- un générateur 12 de flux d'air agencé pour propulser de l'air dans le couloir de ventilation, et
- un support de fixation 13 adapté pour fixer la capsule 0 de parfum dans le couloir de ventilation.

Ces éléments du dispositif sont agencés de sorte que l'air propulsé diffuse le parfum. Le générateur de flux d'air est plus particulièrement une turbine et le flux d'air est préférentiellement légèrement sous pression par rapport à la pression atmosphérique. Typiquement, la surpression de l'air traversant la capsule est de l'ordre de 0,10 à 0,80 inch H2O à 21 °C, soit de l'ordre de 25 à 200 pascals.

La capsule est jetable et remplacée après usage. A cette fin, il est envisagé que le dispositif de diffusion de parfum comprenne des moyens d'insertion et de retrait d'au moins une capsule dans le couloir de ventilation 11 au niveau du support de fixation 13. Ces moyens d'insertion et de retrait sont agencés conjointement ou non avec le support de fixation 13. Par ailleurs, le support de fixation peut aussi bien permettre la fixation d'une capsule ou de plusieurs capsules juxtaposées entre elles comme décrit plus bas.

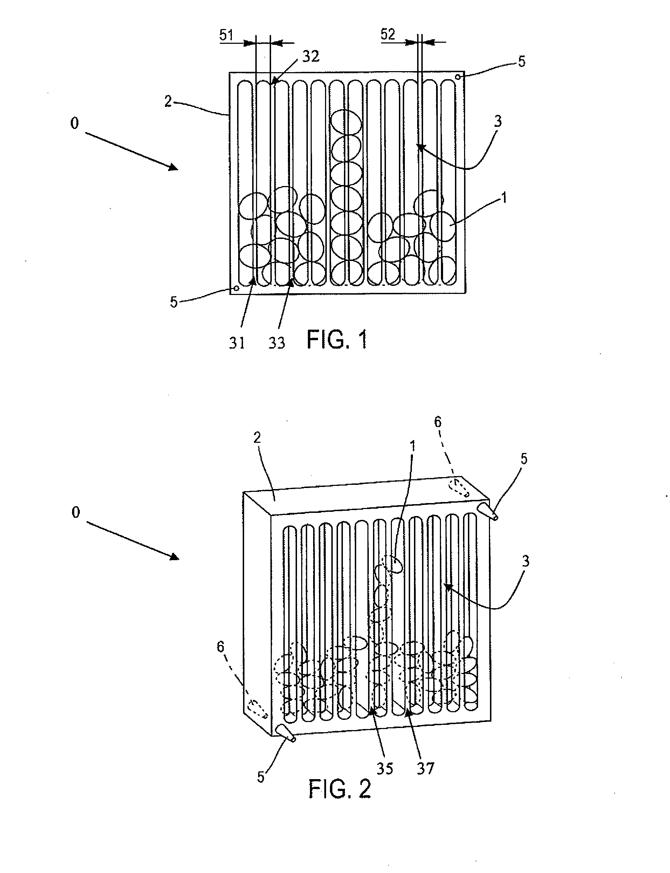

Dans son acceptation la plus large, et comme représenté sur les figures 1 à 3, la capsule 0 de parfum comprend :
- des éléments de substrat 1 dans lesquels est adsorbé un parfum,
- un cadre 2,
- une première grille 3 et une seconde grille 4 fixées dans le cadre 2 en vis-à-vis l'une de l'autre, chacune des grilles 3, 4 comprenant une pluralité de barreaux 31, 32, 33, ...,41,42,43, ....

Les éléments de substrat sont plus particulièrement propres à autoriser la pénétration ou la fixation au moins à leur périphérie de molécules du parfum. En l'état, les éléments de substrat diffusent spontanément en petite quantité les molécules de parfum qu'elles contiennent par évaporation naturelle, ou plus particulièrement par un phénomène de désorption.

Les éléments de substrat sont solides. Ils sont réalisés au moins en partie en un matériau poreux et/ou en un matériau polymère. Les matériaux poreux sont constitués par exemple de céramique ou de bois.

Les éléments de substrat peuvent prendre la forme de sphéroïdes, de parallélépipèdes rhomboédriques ou rectangles, ou de prismatoïdes.

Dans un mode de réalisation préféré, les éléments de substrat sont des billes sphéroïdes de matériau polymère et les molécules de parfum sont adsorbées dans tout le volume de chaque bille. En fonction du matériau polymère utilisé et du parfum, les billes diffusent naturellement les molécules de parfum pendant 6 à 18 mois de manière très peu perceptible. Un exemple de tel matériau consiste en du Pebax®.

A titre d'exemple, les billes en matériau polymère présentent chacune, avant adsorption du parfum, une plus petite dimension égale à 3 mm et une plus grande dimension égale à 4 mm, et, après adsorption du parfum, une plus petite dimension égale à 4 mm et une plus grande dimension égale à 6 mm. Le poids de parfum adsorbé dans chaque bille correspond sensiblement au poids d'une bille avant adsorption du parfum.

Le cadre, les grilles et leurs barreaux sont réalisés en un matériau qui résiste chimiquement au parfum. Par exemple, ce matériau est un plastique, tel qu'un polyamide (PA). Les matériaux plastiques présentent l'avantage d'être généralement peu coûteux.

Le cadre, les grilles et leurs barreaux peuvent également être réalisés en un matériau inerte aux parfums. On entend par un matériau inerte aux parfums un matériau avec lequel les molécules de parfum n'interagissent pas. Un tel matériau ne peut donc pas être imprégné ou contaminé par les parfums. Ledit matériau inerte aux parfums peut être, de manière non limitative, de l'aluminium, un métal tel que le cuivre ou le zinc, du verre ou un matériau traité en surface pour être inerte aux parfums. Il est toutefois à noter que la pollution olfactive de la capsule par le parfum ne pose pas véritablement de problèmes dans la mesure où la capsule en son entier est jetable et remplacée après usage.

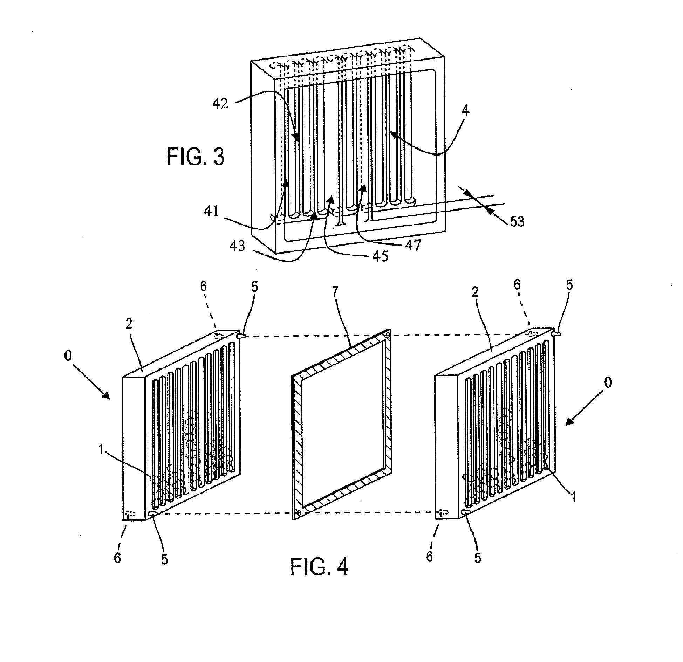

Une des deux grilles, par exemple la grille 3, peut être fixée solidairement au cadre 2, voire former une seule pièce avec le cadre 2, comme cela est illustré sur la figure 3, de façon à ce que cette grille 3 forme avec le cadre 2 un récipient ouvert par la face du cadre 2 destinée à accueillir l'autre grille 4, cette autre grille 4 étant par exemple fixée, de façon amovible ou non amovible, au cadre 2 comme suite au chargement du récipient en éléments de substrat 1.

Le flux d'air traversant la capsule 0 induit un accroissement de la quantité de molécules de parfum diffusées par unité de temps, par rapport à la quantité de molécules de parfum qui seraient évaporées par unité de temps en l'absence de flux d'air. Le phénomène de désorption se conjugue effectivement avec la convection du flux d'air. Plus particulièrement, les molécules de parfum en périphérie des éléments de substrat sont en quelque sorte arrachées au substrat par le flux d'air, et conséquemment les molécules de parfum plus profondément adsorbées dans l'élément de substrat sont attirées vers la périphérie de l'élément pour être arrachées à leur tour, etc.

Selon une particularité essentielle de la capsule 0 et comme illustré sur les figures 1 et 2, les barreaux 31, 32, 33, ..., 41, 42, 43, ... d'une même grille sont espacés entre eux et les grilles 3, 4 sont espacées entre elles, de sorte que la capsule 0 permette le maintien dans le cadre 2 et entre les grilles 3, 4 d'une seule couche d'éléments de substrat 1. La circulation d'air dans le cadre 2 autour des éléments de substrat 1 et des barreaux 31, 32, 33, ..., 41, 42, 43, ... permet la diffusion du parfum.

Les éléments de substrat sont ainsi disposés dans la capsule sur une seule couche, ce qui présente plusieurs avantages.

Premièrement, la surface totale des éléments de substrat au contact de l'air circulant dans le cadre est optimisée, voire maximisée, pour un nombre d'éléments de substrat donné. En effet, moins nombreux sont les points de contact ou moins grandes sont les surfaces de contact entre les éléments de substrat, plus grande est la surface des éléments de substrat léchée par le flux d'air traversant la capsule.

Deuxièmement, la vitesse à laquelle le parfum est désorbé dans l'atmosphère est sensiblement la même pour chaque élément de substrat. Autrement dit, chaque élément de substrat de la couche est exposé au flux d'air pareillement que les autres.

Troisièmement, l'épaisseur de la capsule 0, ou équivalemment du cadre 2, est relativement faible en comparaison de ses autres dimensions ce qui rend la capsule compatible avec des dispositifs 10 de diffusion de parfum compacts. Typiquement, la capsule présente une largeur et une longueur/hauteur de l'ordre de quelques centimètres, et préférentiellement égales à 5 cm, et une épaisseur de l'ordre du centimètre, et préférentiellement égale à 0,8 cm. A titre d'exemple, le couloir de ventilation 11 du dispositif de diffusion de parfum présente une section transverse de largeur et de longueur/hauteur sensiblement plus élevées que celles de la capsule de façon à ce que la capsule puisse être insérée sensiblement sans jeu dans le couloir de ventilation. Typiquement, le dispositif 10 de diffusion de parfum se présente sous la forme d'un parallélépipède de 6 cm de large, 3 cm de profondeur et 6 cm de haut.

Quatrièmement, un flux d'air traversant la capsule est équiréparti sur les éléments de substrat et ainsi une qualité de parfum parfaite est obtenue. En effet, le flux d'air entraîne ainsi, depuis chaque élément de substrat de la couche, une quantité de molécules de parfum quasiment constante sur un intervalle de temps donné et quasiment identique entre les différents éléments de substrat. Cette quantité est plus particulièrement adaptée, lors de la fabrication des éléments de substrat et en fonction du parfum à diffuser, pour créer une ambiance olfactive qui rende compte de la qualité du parfum.

En outre, comme le générateur 12 de flux d'air du dispositif de diffusion de parfum est propre à faire circuler de l'air d'amont en aval du couloir de ventilation 11 au travers de la capsule 0, les avantages suivants sont également atteints.

Premièrement, une diffusion satisfaisante des molécules de parfum dans l'atmosphère par le flux d'air nécessite une pression moindre que celle qui serait requise si des éléments de substrat étaient disposés sur plusieurs couches. En effet, à flux d'air constant en amont de la capsule, la force du flux d'air en sortie du couloir de ventilation, c'est-à-dire en aval de la capsule, est plus grande si les éléments de parfum sont disposés sur une seule couche que s'ils étaient disposés sur plusieurs couches. Les contraintes auxquelles doit satisfaire le générateur de flux d'air en sont d'autant moins élevées. Typiquement, l'utilisation de ventilateurs ou de turbines de faibles puissances, et donc bons marchés et relativement silencieux, est avantageusement suffisante et satisfaisante.

Deuxièmement, le débit en molécules de parfum diffusées est sensiblement invariant, et ainsi, le dosage de la quantité de molécules de parfum diffusées, dans un laps de temps donné et en fonction du volume de la pièce à parfumer, est aisé et précis.

Notamment lorsqu'il est réalisé en un matériau polymère, chaque élément de substrat 1 présente des dimensions qui varient dans le temps depuis des dimensions initiales jusqu'à des dimensions finales plus petites atteintes lorsque le parfum est entièrement désorbé.

Les dimensions initiales des éléments de substrat 1 sont généralement comprises entre 1 mm et 15 mm. Plus particulièrement, la plus petite dimension initiale des billes en matériau polymère est égale à 4 mm et la plus grande dimension initiale des billes en matériau polymère est égale à 6 mm, ceux qui correspond exactement aux dimensions indiquées plus haut des billes en matériau polymère après adsorption du parfum.

Les dimensions finales des éléments de substrat 1 en matériau polymère sont comprises entre un demi et huit dixièmes de leurs dimensions initiales. Plus particulièrement, la plus petite dimension finale des billes en matériau polymère est égale à 3 mm et la plus grande dimension finale des billes en matériau polymère est égale à 4 mm, ceux qui correspond exactement aux dimensions indiquées plus haut des billes en matériau polymère avant adsorption du parfum.

Selon une autre particularité, les première et seconde grilles 3, 4 sont planes et parallèles entre elles, lorsqu'elles sont fixées dans le cadre 2. De plus, les barreaux 31, 32, 33, ..., 41, 42, 43, ... de chaque grille sont rectilignes et parallèles entre eux. En outre, chaque barreau 31, 32, 33, ... de la première grille 3 se trouve en vis-à-vis d'un barreau 41, 42, 43, ... de la seconde grille 4, comme représenté sur la figure 1 où un barreau 31 de la grille 3 cache un barreau 41 de la grille 4.

La capsule est ainsi avantageusement de fabrication industrielle aisée, les formes des grilles et de leurs barreaux étant les plus simples possibles. En outre, le flux d'air traversant la capsule est avantageusement proche d'un flux d'air laminaire. En effet, un barreau de la première grille 3 faisant face à un barreau de la seconde grille 4, les barreaux constituent un moindre obstacle pour le flux d'air traversant la capsule. Ce dernier étant ainsi perturbé au minimum par les barreaux n'est avantageusement pas significativement turbulent, notamment en aval de la capsule.

Selon une autre particularité, et comme illustré sur la figure 1, pour chaque grille 3, 4, la distance 51 entre deux barreaux 31, 32, 33, ..., 41, 42, 43, ... premiers voisins et l'épaisseur 52 de chaque barreau 31, 32, 33, ..., 41, 42, 43, ... sont strictement inférieures à la plus petite dimension, initiale et/ou finale, des éléments de substrat 1. En outre, comme illustré sur la figure 3, l'espacement 53 entre les première et seconde grilles 3, 4 est supérieur à la plus petite dimension initiale des éléments de substrat 1 et inférieur à la plus grande dimension initiale des éléments de substrat 1.

Typiquement, l'épaisseur 52 de chaque barreau est égale à 0,8 mm.

Avantageusement, les éléments de substrat 1 sont ainsi au moins initialement maintenus enserrés, ou du moins coincés, entre les barreaux des grilles 3, 4. En outre, à mesure que leurs dimensions rapetissent, les éléments de substrat 1 deviennent au moins partiellement libres de se mouvoir entre les grilles 3, 4, sans pour autant pouvoir passer entre les barreaux 31, 32, 33, ..., 41, 42, 43, ....

Selon une autre particularité, au moins deux barreaux 31, 32, 33, ..., 41, 42, 43, ... seconds voisins de la première grille 3 et/ou de la seconde grille 4 s'étendent dans l'épaisseur du cadre 2. Par exemple, ces deux barreaux seconds voisins sont les barreaux 45 et 47 illustrés sur la figure 3. Entre ces deux barreaux seconds voisins, une seule rangée d'éléments de substrat 1 de la couche d'éléments de substrat peut être maintenue. Par un premier exemple correspondant à celui représenté sur la figure 3, les deux barreaux 45 et 47 seconds voisins de la grille 4 s'étendent dans l'épaisseur du cadre 2 jusqu'à toucher les barreaux 35 et 37 de l'autre grille 3 (représentés sur la figure 2) qui se trouvent en vis-à-vis. Par un autre exemple non illustré, lesdits barreaux seconds voisins de chaque grille 3 et 4 s'étendent dans l'épaisseur du cadre 2 jusqu'à se toucher au milieu de l'épaisseur du cadre 2.

Il doit être entendu que la seule contrainte est de maintenir les éléments de substrat 1 initialement disposés dans la rangée durant toute la durée d'utilisation de la capsule, c'est-à-dire jusqu'à ce que les éléments de substrat aient atteints leurs dimensions finales. Ainsi, selon le premier exemple ci-dessus, il est autorisé un espacement entre chaque barreau s'étendant dans l'épaisseur du cadre 2 et le barreau de l'autre grille 4 qui se trouve en vis-à-vis, si cet espacement est inférieur à la plus petite dimension finale des éléments de substrat.

Avantageusement, la couche d'éléments de substrat comprend ainsi au moins une rangée d'éléments de substrat. Cette rangée présente préférentiellement un nombre d'éléments de substrat maîtrisé. Lors du chargement industriel des éléments de substrat 1 dans la capsule 0, la maîtrise du nombre d'éléments de substrat est plus aisée si elle concerne uniquement ceux de la rangée, plutôt que tous ceux de la couche.

En outre, les barreaux 31, 32, 33, ..., 41, 42, 43, ... seconds voisins s'étendant dans l'épaisseur du cadre 2 peuvent être disposés verticalement, comme illustré sur la figure 3. De cette façon, les éléments de substrat 1 de la rangée, au nombre de 7 sur l'illustration faite en figure 1, devenant dans le temps plus ou moins libres en mouvement vertical entre lesdits barreaux seconds voisins, se tassent au moins sous l'action de leur poids. La hauteur de la rangée constitue alors un indicateur visuel d'usure de la capsule 0. A titre d'exemple, un segment composé de 10 éléments de substrat 1 sphériques de diamètre égal à 3 mm a une longueur de 30 mm. Si le diamètre de chaque élément de substrat diminue de 20% en moyenne sous l'effet de l'usure conjugué à la pesanteur, la longueur du segment devient égale à 24 mm du moins sous l'effet de la pesanteur. Un des deux barreaux s'étendant dans l'épaisseur du cadre peut présenter un repère indiquant la hauteur de la rangée à laquelle la capsule, parce qu'elle serait vide, devrait être jetée et remplacée par une autre.

Ainsi, l'état d'usure de la capsule est aisément et simplement observable. De plus, l'indicateur visuel d'usure présente une fiabilité certaine du fait qu'il dépend de l'observation non pas d'un seul élément de substrat, mais d'une pluralité d'éléments de substrat (ceux constituant la rangée). En ce sens, la rangée constitue un indicateur moyen d'usure relativement indépendant de la variabilité d'usure qui pourrait être observée entre éléments de substrat pris isolément.

Il est envisagé qu'un poids soit disposé au dessus de la rangée de billes de façon à s'assurer du tassement de la rangée.

Egalement à cette fin, il est envisagé que ce poids soit réalisé en un matériau magnétique et que la capsule comprenne en outre un aimant permanent agencé par exemple dans le cadre 2. Plus particulièrement et à titre d'exemple, l'aimant est propre à attirer ledit poids et est agencé en dessous de la rangée. Selon un autre exemple, l'aimant est propre à repousser ledit poids et est agencé au dessus de la rangée.

Il est également envisagé que la fonction de tassement de la rangée ne soit pas remplie à l'aide d'un poids, qu'il soit ou non magnétique, mais à l'aide d'un ressort. Ce ressort serait fixé par une première extrémité dans le cadre 2 de la capsule 0 au niveau de la rangée, et à l'autre extrémité du ressort serait fixée une butée, de sorte que le ressort soit maintenu, quel que soit l'état d'usure des éléments de substrat composant la rangée, dans un état de compression entre le cadre 2 et la butée et que la rangée soit corrélativement tassée entre la butée et le cadre 2. L'on comprend que cet agencement remplisse avantageusement la fonction de tasser la rangée d'éléments de substrat 1 que celle-ci soit disposée verticalement ou horizontalement.

En outre, il est envisagé d'asservir la puissance du générateur de flux d'air (dans la mesure où celle-ci est variable) à la hauteur de la rangée, de sorte de maintenir un débit de molécules de parfum constant malgré l'usure progressive des billes. Par exemple, entre une hauteur initiale de la rangée et une hauteur finale plus petite, la puissance du générateur de flux d'air est avantageusement progressivement augmentée. Il faut noter toutefois, que la quantité de molécules de parfum arrachées à flux d'air constant est sensiblement invariant par rapport à l'usure des éléments de substrat, dès lors que ceux-ci contiennent encore des molécules de parfum, car cette quantité dépend en plus grande partie de la surface des éléments de substrat léchée par le flux d'air, qui est sensiblement invariante par rapport à l'usure des éléments de substrat.

Par ailleurs, le cadre 2 peut comprendre au moins un élément mâle 5 de fixation sur une face et au moins autant d'éléments femelles 6 de fixation sur l'autre face, chaque élément mâle étant propre à mettre en prise, de façon préférentiellement amovible, l'un quelconque des éléments femelles. De cette façon, plusieurs capsules 0 peuvent être jointes ensemble, leurs cadres se juxtaposant dans la direction de leur épaisseur.

Par exemple, et comme illustré notamment sur les figures 2 et 4, deux éléments mâles de fixation consistant en des doigts de forme tronconiques sont disposés à deux coins opposés d'une des faces du cadre, l'autre face du cadre présentant deux éléments femelles de fixation consistant en des orifices pratiqués à deux coins opposés de ladite autre face. Comme illustré sur la figure 4, cette configuration permet le positionnement et le maintien mécanique des capsules juxtaposées entre elles, les doigts et les orifices ne pouvant coopérer que si les capsules sont précisément en vis-à-vis et orientées dans un même sens et chaque doigt se coinçant dans chaque orifice correspondant, respectivement.

Ainsi, le positionnement et le maintien mécanique entre capsules juxtaposées sont assurés. En outre, la quantité de molécules de parfum diffusées est modulable, notamment en fonction du volume de la pièce à parfumer. Par exemple, si une capsule comprend un nombre d'éléments de substrat tel qu'elle permet de parfumer de façon satisfaisante une pièce de 30 mètres cubes, il suffit de juxtaposer deux capsules de ce type pour parfumer de façon satisfaisante une pièce de 60 mètres cubes, puisqu'ainsi deux fois plus de molécules de parfum sont diffusées dans l'atmosphère. En outre, même si dans les éléments de substrat de chaque capsule est adsorbé le même parfum, il est avantageusement possible de diffuser un mélange de parfums en juxtaposant au moins deux capsules, dans chacune desquelles un parfum différent est adsorbé dans les éléments de substrat.

Cette possibilité de juxtaposer les capsules entre elles reste compatible avec un dispositif de diffusion de parfum compact, c'est-à-dire de faibles dimensions, du fait de la faible épaisseur de la capsule 0.

En outre et comme illustré sur la figure 4, un joint d'étanchéité 7 à l'air peut être disposé au niveau de la jonction entre deux cadres 2 juxtaposés.

Le flux d'air traversant les capsules 0 juxtaposées par leurs cadres 2 est ainsi contraint de traverser chaque capsule 0 de la juxtaposition sans perte de charge du flux d'air qui serait due à des fuites d'air entre les cadres 2 des capsules 0 juxtaposées.

Il est à noter que le joint d'étanchéité 7 peut avantageusement jouer le rôle de l'élément mâle 5 de fixation. Par exemple, le joint d'étanchéité 7 est partiellement inséré dans une première rainure pratiquée dans le pourtour d'une face du cadre 2, une autre partie du joint d'étanchéité 7 faisant saillie par rapport à la face du cadre 2, et l'autre face de la capsule comprend une seconde rainure équivalente à la première mais n'accueillant pas de joint d'étanchéité 7. Selon cet exemple, une première capsule peut être juxtaposée à une autre par insertion de la partie du joint d'étanchéité 7 faisant saillie dans la rainure pratiquée dans le pourtour de la face du cadre 2 de l'autre capsule.

Dans les revendications, le mot « comprenant » n'exclue pas d'autres éléments et l'article indéfini « un/une » n'exclue pas une pluralité.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes.

## Revendications

1. Capsule (0) de parfum comprenant :
- des éléments de substrat (1) dans lesquels est adsorbé un parfum,
- un cadre (2),
- une première grille (3) et une seconde grille (4) fixées dans le cadre (2) en vis-à-vis l'une de l'autre, chacune des grilles (3, 4) comprenant une pluralité de barreaux (31, 32, 33, ...,41,42,43, ...),
les barreaux (31, 32, 33, ..., 41, 42, 43, ...) d'une même grille étant espacés entre eux et les grilles (3, 4) étant espacées entre elles, de sorte que la capsule (0) permette le maintien dans le cadre (2) et entre les grilles (3, 4) d'une seule couche d'éléments de substrat (1), et une circulation d'air dans le cadre (2) autour des éléments de substrat (1) et des barreaux (31, 32, 33, ..., 41, 42, 43, ...).

2. Capsule (0) de parfum selon la revendication 1, **caractérisé en ce que** chaque élément de substrat (1) présente des dimensions qui varient dans le temps depuis des dimensions initiales jusqu'à des dimensions finales plus petites atteintes lorsque le parfum est entièrement désorbé.

3. Capsule (0) de parfum selon la revendication 1 ou la revendication 2, **caractérisé en ce que** :
- les première et seconde grilles (3, 4) sont planes et parallèles entre elles, et
- les barreaux (31, 32, 33, ..., 41, 42, 43, ...) de chaque grille sont rectilignes et parallèles entre eux,
de sorte que chaque barreau (31, 32, 33, ...) de la première grille (3) se trouve en vis-à-vis d'un barreau (41, 42, 43, ...) de la seconde grille (4).

4. Capsule (0) de parfum selon la revendication 2 ou la revendication 3, **caractérisé en ce que**, pour chaque grille (3, 4), la distance (51) entre deux barreaux (31, 32, 33, ..., 41, 42, 43, ...) premiers voisins et l'épaisseur (52) de chaque barreau (31, 32, 33, ...,41, 42, 43, ...) sont strictement inférieures à la plus petite dimension des éléments de substrat (1), et **en ce que** l'espacement (53) entre les première et seconde grilles (3, 4) est supérieur à la plus petite dimension initiale des éléments de substrat (1) et inférieur à la plus grande dimension initiale des éléments de substrat (1).

5. Capsule (0) de parfum selon l'une des revendications précédentes, **caractérisé en ce qu'**au moins deux barreaux (31, 32, 33, ..., 41, 42, 43, ...) seconds voisins de la première grille (3) et/ou de la seconde grille (4) s'étendent dans l'épaisseur du cadre (2), de sorte qu'une seule rangée d'éléments de substrat (1) de la couche d'éléments de substrat soit maintenue entre les deux barreaux seconds voisins s'étendant dans l'épaisseur du cadre (2).

6. Capsule (0) de parfum selon la revendication précédente, **caractérisé en ce que** les barreaux (31, 32, 33, ..., 41, 42, 43, ...) seconds voisins s'étendant dans l'épaisseur du cadre (2) sont disposés verticalement de sorte que, les éléments de substrat (1) de la rangée se tassant dans le temps au moins sous l'action de leur poids, la hauteur de la rangée constitue un indicateur visuel d'usure de la capsule (0).

7. Capsule (0) de parfum selon l'une des revendications 5 et 6, **caractérisé en ce qu'**un poids est disposé au dessus de la rangée de billes.

8. Capsule (0) de parfum selon la revendication 5, **caractérisé en ce qu'**elle comprend en outre un ressort fixé par une première extrémité dans le cadre (2) au niveau de la rangée et une butée fixée à l'autre extrémité du ressort, de sorte que le ressort soit maintenu, quel que soit l'état d'usure des éléments de substrat (1), dans un état de compression entre le cadre et la butée.

9. Capsule (0) de parfum selon l'une des revendications précédentes, **caractérisé en ce que** le cadre (2) comprend au moins un élément mâle (5) de fixation sur une face et au moins autant d'éléments femelles (6) de fixation sur l'autre face, chaque élément mâle étant propre à mettre en prise l'un quelconque des éléments femelles, de sorte que plusieurs capsules (0) puissent être jointes ensemble, leurs cadres se juxtaposant dans la direction de leur épaisseur.

10. Capsule (0) de parfum selon la revendication précédente, **caractérisé en ce qu'**un joint d'étanchéité (7) à l'air est disposé au niveau de la jonction entre deux cadres juxtaposés.

11. Dispositif (10) de diffusion de parfum comprenant :
- une capsule (0) selon l'une quelconque des revendications précédentes,
- un couloir de ventilation (11),
- un générateur (12) de flux d'air agencé pour propulser de l'air dans le couloir de ventilation, et
- un support de fixation (13) adapté pour fixer la capsule (0) de parfum dans le couloir de ventilation,
de sorte que l'air propulsé diffuse le parfum.

## Patentansprüche

1. Parfümkapsel (0), umfassend:
- Substratelemente (1), in denen ein Parfüm adsorbiert ist,
- einen Rahmen (2),
- ein erstes Gitter (3) und ein zweites Gitter (4), die im Rahmen (2) einander gegenüber befestigt sind, wobei jedes der Gitter (3, 4) eine Vielzahl von Stangen (31, 32, 33, ..., 41, 42, 43, ...) umfasst,
wobei die Stangen (31, 32, 33, ..., 41, 42, 43, ...) eines und desselben Gitters untereinander beabstandet sind und die Gitter (3, 4) untereinander derart beabstandet sind, dass die Kapsel (0) das Festhalten einer einzigen Schicht von Substratelementen (1) im Rahmen (2) und zwischen den Gittern (3, 4) und eine Luftzirkulation im Rahmen (2) rund um die Substratelemente (1) und die Stangen (31, 32, 33, ..., 41, 42, 43, ...) zulässt.

2. Parfümkapsel (0) gemäß Anspruch 1, **dadurch gekennzeichnet, dass** jedes Substratelement (1) Abmessungen aufweist, die im Zeitverlauf von den ursprünglichen Abmessungen bis zu den endgültigen kleineren Abmessungen, die erreicht werden, wenn das Parfüm komplett adsorbiert ist, variieren.

3. Parfümkapsel (0) gemäß Anspruch 1 oder Anspruch 2, **dadurch gekennzeichnet, dass**:
- das erste und zweite Gitter (3, 4) eben und untereinander parallel sind und
- die Stangen (31, 32, 33, ..., 41, 42, 43, ...) jedes Gitters geradlinig und untereinander parallel sind,
derart, dass jede Stange (31, 32, 33, ...) des ersten Gitters (3) sich gegenüber einer Stange (41, 42, 43, ...) des zweiten Gitters (4) befindet.

4. Parfümkapsel (0) gemäß Anspruch 2 oder Anspruch 3, **dadurch gekennzeichnet, dass** für jedes Gitter (3, 4) die Entfernung (51) zwischen zwei ersten, benachbarten Stangen (31, 32, 33, ..., 41, 42, 43, ...) und der Dicke (52) jeder Stange (31, 32, 33, ..., 41, 42, 43, ...) strikt kleiner ist als die kleinste Abmessung der Substratelemente (1) und dass die Beabstandung (53) zwischen dem ersten und zweiten Gitter (3, 4) größer ist als die kleinste ursprüngliche Abmessung der Substratelemente (1) und kleiner als die größte ursprüngliche Abmessung der Substratelemente (1).

5. Parfümkapsel (0) gemäß einem der voranstehenden Ansprüche, **dadurch gekennzeichnet, dass** wenigstens zwei zweite, benachbarte Stangen (31, 32, 33, ..., 41, 42, 43, ...) des ersten Gittes (3) und /oder des zweiten Gitters (4) sich in der Dicke des Rahmens (2) derart erstrecken, dass eine einzige Reihe von Substratelementen (1) der Schicht von Substratelementen zwischen den zwei zweiten benachbarten Stangen gehalten wird, die sich in der Dicke des Rahmens (2) erstrecken.

6. Parfümkapsel (0) gemäß dem voranstehenden Anspruch, **dadurch gekennzeichnet, dass** die zweiten, benachbarten Stangen (31, 32, 33, ..., 41, 42, 43, ...), die sich in der Dicke des Rahmens (2) erstrecken, derart vertikal angeordnet sind, dass die Substratelemente (1) der Reihe sich im Zeitverlauf wenigstens unter der Wirkung ihres Gewichts setzen, die Höhe der Reihe stellt einen visuellen Nutzungsindikator der Kapsel (0) dar.

7. Parfümkapsel (0) gemäß Anspruch 5 und 6, **dadurch gekennzeichnet, dass** oberhalb der Kugelreihe ein Gewicht angeordnet ist.

8. Parfümkapsel (0) gemäß Anspruch 5, **dadurch gekennzeichnet, dass** sie darüber hinaus eine Feder umfasst, die durch ein erstes Ende im Rahmen (2) auf der Höhe der Reihe befestigt ist, und einen Anschlag, der am anderen Ende der Feder derart befestigt ist, dass die Feder unabhängig von dem Abnutzungszustand der Substratelemente (1) in einem Komprimierungszustand zwischen dem Rahmen und dem Anschlag gehalten wird, umfasst.

9. Parfümkapsel (0) gemäß einem der voranstehenden Ansprüche, **dadurch gekennzeichnet, dass** der Rahmen (2) wenigstens ein männliches Befestigungselement (5) auf einer Seite und wenigstens ebenso viele weibliche Befestigungselemente (6) auf der anderen Seite umfasst, wobei jedes männliche Element geeignet ist, in jedes beliebige weibliche Element derart einzugreifen, dass mehrere Kapseln (0) untereinander verbunden werden können, wobei ihre Rahmen in der Richtung ihrer Dicke nebeneinander liegen.

10. Parfümkapsel (0) gemäß dem voranstehenden Anspruch, **dadurch gekennzeichnet, dass** eine luftdichte Dichtung (7) auf der Höhe der Nahtstelle zwischen zwei nebeneinander liegenden Rahmen angeordnet ist.

11. Parfümverteilervorrichtung (10), umfassend:
- eine Kapsel (0) gemäß einem der voranstehenden Ansprüche,
- einen Belüftungsgang (11),
- einen Luftstromgenerator (12), der angeordnet ist, um Luft in den Belüftungsgang auszustoßen, und
- einen Befestigungsträger (13), der angepasst ist, um die Parfümkapsel (0) in dem Belüftungsgang derart zu befestigen, dass die ausgestoßene Luft das Parfüm verteilt.

## Claims

1. A fragrance capsule (0) comprising:
- substrate elements (1) wherein a fragrance is adsorbed,
- a frame (2),
- a first grid (3) and a second grid (4) attached in the frame (2) opposite one another, with each of the grids (3, 4) comprising a plurality of bars (31, 32, 33, ..., 41, 42, 43,...),
with the bars (31, 32, 33, ..., 41, 42, 43,...) of the same grid being spaced from each other and the grids (3, 4) being spaced from each other, so that the capsule (0) makes it possible to hold a single layer of substrate elements (1) within the frame (2) and between the grids (3, 4), and enables air to circulate in the frame (2), around the substrate elements (1) and the bars (31, 32, 33, ..., 41, 42, 43,...).

2. A fragrance capsule (0) according to claim 1, **characterized in that** each substrate element (1) has dimensions which vary over time from initial dimensions to smaller final dimensions reached when the fragrance is fully desorbed.

3. A fragrance capsule (0) according to claim 1 or claim 2, **characterized in that**:
- the first and second grids (3, 4) are flat and parallel to each other, and
- the bars (31, 32, 33, ..., 41, 42, 43,...) of each grid are rectilinear and parallel to each other,
so that each bar (31, 32, 33,...) of the first grid (3) is located opposite a bar (41, 42, 43,...) of the second grid (4).

4. A fragrance capsule (0) according to claim 2 or claim 3, **characterized in that**, for each grid (3, 4), the distance (51) between two first neighbouring bars (31, 32, 33, ..., 41, 42, 43,...) and the thickness (52) of each bar (31, 32, 33, ..., 41, 42, 43,...) are strictly smaller than the smallest dimension of the substrate elements (1), and **in that** the spacing (53) between the first and second grids (3, 4) is greater than the smallest initial dimension of the substrate elements (1) and smaller than the largest initial dimension of the substrate elements (1).

5. A fragrance capsule (0) according to one of the preceding claims, **characterized in that** at least two second neighbouring bars (31, 32, 33, ..., 41, 42, 43,...) of the first grid (3) and/or of the second grid (4) extend into the thickness of the frame (2), so that only one row of substrate elements (1) of the layer of substrate elements is held between the two second neighbouring bars extending into the thickness of the frame (2).

6. A fragrance capsule (0) according to the preceding claim, **characterized in that** the second neighbouring bars (31, 32, 33, ..., 41, 42, 43,...) extending into the thickness of the frame (2) are arranged vertically so that, although the substrate elements (1) of the row settle over time, at least under the action of their own weight, the height of the row provides a visual indicator of the wearing of the capsule (0).

7. A fragrance capsule (0) according to one of Claims 5 and 6, **characterized in that** a weight is arranged above the row of balls.

8. A fragrance capsule (0) according to claim 5, **characterized in that** it further comprises a spring attached at one end in the frame (2), at the row level and a stop attached at the other end of the spring, so that the spring is held, whatever the worn state of the substrate elements (1), in a compressed state between the frame and the stop.

9. A fragrance capsule (0) according to one of the preceding claims, **characterized in that** the frame (2) comprises at least one male attaching element (5) on one face and at least as many female attaching elements (6) on the other face, with each male element being adapted to engage into any one of the female elements, so that several capsules (0) can be joined together, with the frames thereof being juxtaposed in the direction of the thickness thereof.

10. A fragrance capsule (0) according to the preceding claim, **characterized in that** an air seal (7) is positioned at the junction between two juxtaposed frames.

11. A fragrance diffusing device (10) comprising:
- a capsule (0) according to any one of the preceding claims,
- a ventilation passage (11),
- an air flow generator (12) so arranged as to propel air into the ventilation passage, and
- a mounting bracket (13) adapted for securing the fragrance capsule (0) in the ventilation passage,
so that the propelled air diffuses the fragrance.
